# EUROPEAN PATENT APPLICATION

(11) **EP 1 405 871 A1**
(43) Date of publication of application: **07.04.2004**
(21) Application number: 02730784.2
(22) Date of filing: 30.05.2002
(51) Int. Cl.: C08G 85/00

(54) **METHOD OF BONDING SUBSTANCE TO BE INCORPORATED TO POLYMER TERMINAL**

(30) Priority: 30.05.2001 JP 2001161788
(71) Applicant: Mitsubishi Kagaku Iatron, Inc., Tokyo 101-0031 (JP); IATRON LABORATORIES, INC., Chiyoda-ku Tokyo 101-0031 (JP); Kataoka, Kazunori, Nakano-ku, Tokyo 165-0031 (JP)
(72) Inventor: KATAOKA, Kazunori, Nakano-ku, Tokyo 165-0031 (JP); 3NAGASAKI, Yukio, Moriya-shi, Ibaraki 302-0128 (JP); SHIBATA, Naoya, Matsudo-shi, Chiba 271-0051 (JP); HOSHINO, Nobuhiro c/o IATRON LABORATORIES, INC., Tokyo 101-0031 (JP)
(74) Representative: VOSSIUS & PARTNER
(86) International application number: PCT/JP2002/005272
(87) International publication number: WO 2002/096977

(57) **Abstract**

A method of bonding a substance to be incorporated into a free terminal of a water-soluble polymer compound chain, characterized by reacting a reactive functional group present at the free terminal of the water-soluble polymer compound chain which is bonded at a binding terminal thereof in the manner of bristles of a brush onto a surface of a base material; with the substance to be incorporated capable of reacting with the reactive functional group; in the presence of a water-soluble polymer compound which promotes the bonding, is disclosed.

According to the method of the present invention, when reactive functional groups present at the terminals of water-soluble polymer compound chains which are bonded in the manner of bristles of a brush on the surface of a base material are reacted with substances to be incorporated capable of reacting with the functional group, the substances can be bonded to the polymer compound terminals at a high efficiency.

## Description

### TECHNICAL FIELD

The present invention relates to a method for bonding a substance to be incorporated into the terminal of a polymer compound. The present invention, for example, raises the bonding efficiency between a polyethyleneglycol (PEG), which is used as a functional polymer, and a substance to be incorporated having a desired function, in a reaction of the substance to be incorporated, with a base material having the PEG in the manner of bristles of a brush on the surface thereof, and thus provides highly-reactive functional materials at a high yield.

### BACKGROUND ART

As one application of polyethyleneglycols (PEGs), a method is known of amplifying signals in assay systems by using the capability of accelerating agglutination due to antigen-antibody reactions. It is assumed that the addition of several percentages of PEG into a solution of an analyte antigen and an antiserum or antibody-bonding particle brings about the destruction of the bonded water in these proteins, essential for the preservation of the structure thereof, thus accelerating precipitation.

Alternatively, much research has been carried out into improving the thermostability and organic solvent resistance of proteins (for example, enzymes, antibodies, or the like) by modifying the proteins with straight chain PEGs, as another application of PEGs, and these PEG-modified proteins have been applied in a variety of industrial fields. For example, a modified cholesterol oxidase which is stable and exhibits an activity in toluene was prepared by bonding a PEG having the OH group thereof activated with cyanuric chloride to surface amino groups of the enzyme.

In addition, such a PEG modification is known to be effective in suppressing rejection reactions of a iving body against an enzyme, hormone, or the like administered as a medicine, or in suppressing a deterioration in effect of the medicine due to a rapid decomposition thereof. For example, it has been reported that a PEG modified interleukin-2, a cytokine, significantly prolongs the life of mice which are injected intravenously, because the blood level of the biological activity was maintained at a certain level for a period longer than that of the unmodified interleukin-2.

Recently, it was also made clear that the PEG coating is effective as a technique for a surface treatment of the materials that are in contact with a living body or with biological samples such as blood and the like. For example, the presence of PEG formed in the manner of bristles of a brush on the surface of a base material drastically inhibits the adsorption of proteins and cells on the surface thereof, and there are great expectations of an application of such a coating to the surface of catheters, artificial organs, or diagnostic devices [for example, Ohtsuka, Nagasaki, and Kataoka et al., Trans. Mater. Res. Soe. Jpn., 25 (4), 895-898 (2000); and Leckband et al., J. Biomater. Sci., Polym. Ed., 10 (10), 1125-1147 (1999)].

The present inventors have, for a long time, developed methods for preparing a variety of polyethyleneglycol (PEG) derivatives having functional groups. During the course of this development, the inventors found that it is possible to prepare nanoparticles having a PEG brush structure on the surface thereof, by a dispersion polymerization of a vinyl monomer (for example, styrene, methacrylic acid methyl, isoprene, or the like) in a dispersion liquid containing a PEG macromonomer having a polymerizable functional group at the opposite terminal and a block-copolymeric macromonomer having polylactic acid segments as dispersants. The nanoparticles thus obtained are high-performance reactive core-shell nanoparticles, as the surface layer PEG brush thereof contains functional groups of, for example, an' aldehyde or amino group at its terminal.

The particles thus obtained are protected from a nonspecific adsorption of proteins and the like by the PEG brush structure present' on the surface thereof. By introducing a ligand at the PEG terminal, the particles provide high-performance particles that detect, at a high sensitivity, specific interactions such as ligand-receptor interactions including antigen-antibody reactions.

In this manner, PEGs and proteins in an aqueous solution exhibit special interactions, the proper use of which will lead to many beneficial applications of PEGS.

As described above, although it is known that the materials having a PEG on the surface thereof exhibit smaller interference with biological components, and the occurence of phenomena such as the adsorption of protein are fewer, almost no attempt has been made to use PEGs in applications wherein the a physiologically active substance is intentionally bonded to the PEG surface, allowing them to exhibit the physiological property thereof, and other nonspecific reactions are suppressed. Accordingly, there' is no known method of bonding efficiently a physiologically active substance, such as an antigen, antibody, enzyme, hormone, DNA, bacteria, or the like, to the PEG surface. The present inventors have conducted experiments into preparing functional materials having a high specificity by bonding proteins to aldehyde groups present at the PEG terminals, and found that the yield thereof is extremely low. Accordingly, the inventors determined that it was necessary to investigate the optimal condition therefor, as there was no effective method available in literature or in past patent publications.

### DISCLOSURE OF THE INVENTION

An object of the present invention is to provide a method in which, when reactive functional groups present at the terminals of water-soluble polymer compounds which are bonded in the manner of bristles of a brush on the surface of a base material (for example, a polymer brush) are reacted with substances to be incorporated capable of reacting with the functional group, the substances can be bonded to the polymer compound terminals at a high efficiency.

The object above is accomplished by a method of bonding a substance to be incorporated to a free terminal of a water-soluble polymer compound chain, according to the present invention, characterized by reacting
(1) a reactive functional group present at the free terminal of the water-soluble polymer compound chain which is bonded at a binding terminal thereof in the manner of bristles of a brush onto a surface of a base material; with
(2) the substance to be incorporated capable of reacting with the reactive functional group;
in the presence of a water-soluble polymer compound which promotes the bonding.

In addition, the present invention relates to a method of bonding a substance to be incorporated to a free terminal of a water-soluble polymer compound chain, characterized by reacting
(1) a core-shell particle consisting of
   (a) a core portion substantially made of a water-insoluble polymer compound, and
   (b) a shell portion substantially made of a water-soluble. polymer compound having a reactive functional group, and covering a surface of the core portion in the manner of bristles of a brush,
   the core portion and the shell portion being, as a whole, a block copolymer of a water-insoluble polymer and a water-soluble polymer; with
(2) the substance to be incorporated capable of reacting with the reactive functional group;
in the presence of a water-soluble polymer compound which promotes the bonding.

### BRIEF DESCRIPTION OF THE DRAWINGS

Figure 1 shows a ¹H-NMR spectrum of the block copolymer prepared in EXAMPLE (1).
Figure 2 shows a scanning electron microscope (SEM) photograph of the PEG-coated particles having aldehyde terminals obtained in EXAMPLE 1(2).
Figure 3 shows an SEM photograph of the PEG-coated particles having aldehyde terminals for comparison obtained in EXAMPLE 1(2).

### BEST MODE FOR CARRYING OUT THE INVENTION

Hereinafter, the present invention will be described in detail.

In the method of the present invention, the water-soluble polymer compound chain that is bonded in the manner of bristles of a brush on the surface of a base material is not particularly limited, so long as it is a straight chain polymer compound that can bind to the surface of the base material at one terminal (binding terminal) and has a reactive functional group or a group to which a reactive functional group can be introduced at the other terminal (free terminal) and can be arranged into the shape of bristles of a brush covering the surface of the base material. The state wherein the "water-soluble polymer compound chains are bonded in the manner of bristles of a brush on the surface of a base material" as used herein means a state wherein each of the straight chain water-soluble polymer compounds bonds to the surface of the base material at the binding terminal and each of the free terminals thereof is projected like bristles or rods from the surface into the reaction solution in which at least the water-soluble polymer compound chain and an substance to be incorporated (for example, antigen or antibody) are reacted.

The water-soluble polymer compound chain may be, for example, polyethyleneglycol (PEG), polyvinylalcohol, polyvinylpyrrolidone, polyamino acid, polyacrylic acid, polydimethylaminoethyl methacrylate, polyallylamine, or the like, and preferably PEG or polyvinyl alcohol.

In the present invention, for the water-soluble polymer compound chains that bond to the surface of a base material in the manner of bristles of a brush, each of the water-soluble polymer compound chains may be substantially formed from only one of the water-soluble polymer compounds, or from a combination of two or more mutually different water-insoluble polymer compounds.

The reactive functional group present at the free terminal of the water-soluble polymer compound chain may be present at one terminal (free terminal) of the water-soluble polymer compound chain before another terminal (binding terminal) thereof is bound. to the surface of a base material, or alternatively may be introduced, after one terminal (binding terminal) of the water-soluble polymer compound chain is bound to the surface of a base material, to another terminal (free terminal). These reactive functional groups are not particularly limited, so long as the functional groups are all stable in water (or an aqueous solvent) and can react with a substance to be introduced [for example, physiologically active substance (for example, antibody, enzyme, or DNA)], and examples thereof include, aldehyde, carboxyl, mercapto, amino, maleimide, vinylsulfone, and methanesulfonyl groups, and preferably aldehyde, amino, carboxyl, and maleimide groups.

The base material (i.e., substrate) as used herein is not particularly limited, so long as it is a base material on which the water-soluble polymer compound chains are bonded in the manner of bristles of a brush, and examples thereof are water-insoluble carriers including metals (for example, gold, silver, aluminum and the like), silica, glass, oxides (for example, titanium oxide and the like), plastics (for example, latex particles), rubbers, woods, polymer micelles, gels, and the like. In addition, the shape of the base material is not particularly limited, and may be, for example, particular, plate-like, tubular, or the like. In the case of a tubular base material, the base material may have the water-soluble polymer compound chain built on the internal and/or external surface thereof in the manner of bristles of a brush. Further, the shape of the surface of base material having the water-soluble polymer compound chains in the manner of bristles of a brush is also not particularly limited, and may be, for example, a flat, curved, of spherical surface, or the like. The base material is preferably a latex particle or polymer micelle.

Typical examples of the polymer micelles used as the base material are the core portion of known core-shell particles. Generally, the core-shell particle consists of (1) a core portion mainly made of a water-insoluble polymer and (2) a shell portion, which covers the surface of the core portion in the manner of bristles of a brush, mainly made of a water-soluble polymer having a reactive functional group. The core portion of the core-shell particle corresponds to the base material in the method of the present invention, while the shell portion of the core-shell particle corresponds to the water-soluble polymer compound chain in the method of the present invention.

A variety of known processes may be applied to the method of bonding the water-soluble polymer compound chains in the manner of bristles of a brush on the base material surface, i.e., the method of forming a polymer compound brush. Known methods of forming the polymer compound brush include, for example, copolymerization of macromonomers, adsorption with block polymers, immobilization of terminal reactive oligomers onto the surface of base material, ionic interaction, or bonding of SH-terminal polymers onto metal surface, and the like.

For example, when the base material is the core portion of a core-shell particle, a variety of known processes may be used as the method for bonding the water-soluble polymer compound chains in the manner of bristles of a brush on the base material surface, i.e., the method for preparing the core-shell particles. The known processes include, for example:
(1) an emulsion method wherein particles are prepared by mixing (a) a block copolymer (hydrophilic/hydrophobic block copolymer) consisting of a hydrophilic segment containing a reactive functional group and a hydrophobic segment bound to each other, and (b) a hydrophobic polymer;
(2) a dispersion polymerization method wherein a hydrophobic monomer is polymerized using a water-soluble polymeric macromonomer having a reactive functional group as a dispersant; or
(3) a method of introducing brush-like bristles of a water-soluble polymer on the surface of a hydrogel particle.

As the method for preparing the hydrophilic/hydrophobic block copolymer used in the above emulsion method (1) and the method for preparing the water-soluble polymeric macromonomer having a reactive functional group used in the above dispersion method (2), for example, the method developed by the present inventors and the co-developers (for example, WO 96/33233, WO 99/571743, or Japanese Unexamined Patent Publication (Kokai) No. 11-322917) may be used.

Compounds that may be used as the hydrophilic/hydrophobic block copolymer or water-soluble polymeric macromonomer may be, for example, the heterotelechelic block copolymers described in WO 96/33233 of the formula (IA): 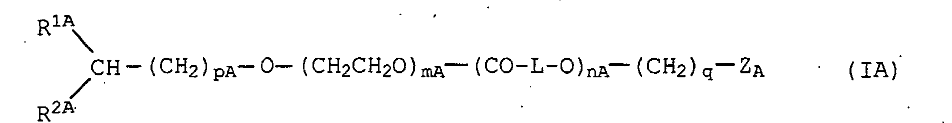 [wherein, R^{1A} and R^{2A} are independently an alkoxy group having 1 to 10 carbon atoms, an aryloxy group, or an aryl(alkyloxy having 1 to 3 carbon atoms) group; R^{1A} and R^{2A} together form an ethylenedioxy group, which may be substituted with an alkyl group having 1 to 6 carbon atoms, of the formula :

-O-CH(R')-CH-O-

(wherein, R' is a hydrogen atom or an alkyl group having 1 to 6 carbon atoms); or
R^{1A} and R^{2A} together form oxy (=O); and
L is a divalent group of the formula:

-CH(R^{3A})-O-CO-CH(R^{4A})-

or

-(CH₂)ᵣ-,

wherein R^{3A} and R^{4A} are independently a hydrogen atom, an alkyl group having 1 to 10 carbon atoms, an aryl group, or an aryl-alkyloxy group having 1 to 3 carbon atoms; r is an integer of 2 to 5; m_{A} is an integer of 2 to 10,000; n_{A} is an integer of 2 to 10,000; p_{A} is an integer of 1 to 5; q is an integer of 0 or 1 to 20; and Z_{A} is a hydrogen atom, an alkali metal, an acetyl, acryloyl, methacroyl, cinnamoyl, p-toluenesulfonyl, 2-mercaptopropionyl, 2-aminopropionyl, allyl, or vinylbenzyl group when q is 0, and an alkoxycarbonyl group having 1 to 6 carbon atoms, a carboxylmercapto or amino group when q is an integer of 1 to 20]. The heterotelechelic block copolymers of the formula (IA) may be prepared, for example, by the method described in WO 96/33233.

In addition, the compounds that may be used as the hydrophilic/hydrophobic block copolymer or water-soluble polymeric macromonomer may be, for example, the polyoxyethylene derivative described in WO 99/571743 of the formula (IB): 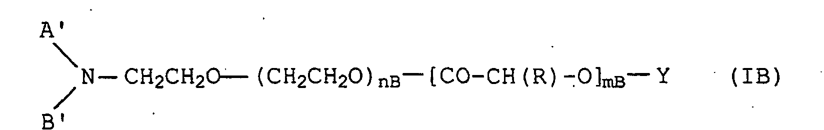 (wherein, A' and B' are independently an amino protecting group of an organic silyl type, or amino protecting groups of an organic silyl type that may form together with the nitrogen atom to which. A' and B' bind a 4- to 7-membered disila-azacyclo heterocyclic ring;
Y is a hydrogen atom, an alkali metal, or an organic group which can be introduced by a suitable reaction replacing the alkali metal; R is a hydrogen atom or an alkyl having 1 to 6 carbon atoms; n_{B} is an integer of 1 to 20,000; and m_{B} is an integer of 0 to 20,000). The polyoxyethylene derivative of the formula (IB) may be, for example, prepared by the method described in WO 99/571743.

The compounds that can be used as the hydrophilic/hydrophobic block copolymer or water-soluble polymeric macromonomer further include, for example, compounds containing an organic silyl sulfide group and polyoxyethylene derivatives described in Japanese Unexamined Patent Publication (Kokai) No. 11-322917 of the formula (IC) : 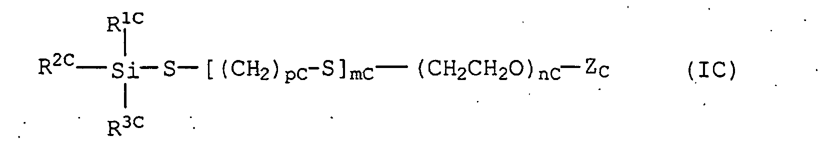 (wherein, R^{1C}, R^{2C}, and R^{3C} are independently a straight chain or branched alkyl group or an aralkyl group; Z_{C} is a functional group selected from the group consisting of a hydrogen atom, acryloyl, methacryloyl, vinylbenzyl, allyl, para-toluenesulfonyl groups, a mono- or di-lower alkyl substituted amino group, an alkyl group having a carboxyl group or the ester group thereof, an alkyl group having an aldehyde group or the acetal group thereof, and an alkali metal; m_{C} is 0 or 1; n_{C} is an integer of 0 to 20,000; and, p_{C} is a positive integer of 2 or 3; with the proviso that m_{C} and n_{C} are not 0 at the same time). The compounds containing an organic silylsulfide group or polyoxyethylene derivatives represented by formula (IC) can be prepared, for example, by the method described in Japanese Unexamined Patent Publication (Kokai) No: 11-322917.

According to the above emulsion method (1) for preparing core-shell particles, a core-shell particle can be prepared by mixing the compound of the formula (IA), (IB), or (IC) (i.e., hydrophilic/hydrophobic block copolymer) and a hydrophobic polymer.

According to the above dispersion method (2) for preparing core-shell particles, a core-shell particle can be prepared by polymerizing a hydrophobic monomer using the compound of the formula (IA), (IB), or (IC) (i.e., water-soluble polymeric macromonomer) as a dispersant.

In the method of the present invention, the substance to be incorporated that can be bonded to the terminal of the water-soluble polymer compound chain is not particularly limited, so long as it can react with the reactive functional group present at the terminal of the water-soluble polymer compound chain, and examples thereof are physiologically active substances such as proteins (for 'example, antibodies and enzymes), nucleic acids (for example, DNAs and RNAs), and cells.

In the method of the present invention, the water-soluble polymer compound which promotes the bonding, i.e., the catalytic water-soluble polymer compound, to be added to the reaction solution is not particularly limited, so long as it is a water-soluble polymer and does not react with the substance to be incorporated, and examples thereof include PEG, polyvinyl alcohol, polyvinylpyrrolidone, polyamino acid, polyacrylic acid, polydimethylaminoethyl methacrylate, polyallylamine, and the like, and preferably PEG and polyvinyl alcohol.

In the method of the present invention, when the substance to be incorporated is reacted with the reactive functional group present at the terminal of the water-soluble polymer compound chain bonded in the manner of bristles of a brush on the surface of the base material, any known reaction conditions may be used as long as the reaction is conducted in the presence of the catalytic water-soluble polymer compound.

The known reaction conditions can be appropriately determined by those in the art in accordance with the kind of reactive functional group and substance to be incorporated. For example, when the reactive functional group is a carboxyl group, the reaction can be carried out by condensation with amino groups present in biological molecules using a condensing agent (for example, carbodiimides or the like). Alternatively, the carboxyl group may be activated by a reaction with succinimide, maleimide, or the like, and then mixed and reacted with the biological molecules.

In the method of the present invention, the concentration of the catalytic water-soluble polymer compound in the reaction solution is not particularly limited, and may be appropriately decided according to the kinds of the reactive functional group, the substance to be incorporated, and/or the catalytic water-soluble polymer compound used. For example, when the reactive functional group is an aldehyde group, the substance to be incorporated is a protein [particularly, bovine serum albumin (BSA)], and the catalytic water-soluble polymer compound is PEG (particularly, PEG 6000), the PEG may be used at a concentration of 0.1 to 6%.

### (Mode of Operation)

In the method of the present invention, it is not currently clear why the substance to be incorporated can be bonded at high efficiency to the free terminal of the water-soluble polymer compound chain which is bonded in the manner of bristles of a brush onto the base material surface, but an assumption is provided below. Namely, when the catalytic water-soluble polymer compound is not present in the reaction system wherein the reactive functional group at the free terminal of the water-soluble polymer compound chain (particularly, PEG chain) reacts with the substance to be incorporated (particularly, protein), the substance to be incorporated and the water-soluble polymer compound chain repel each other, reducing the bonding efficiency. When the catalytic water-soluble polymer compound (particularly, PEG) is added to the system, the mutual repelling force generated between the substance to be incorporated and the catalytic water-soluble polymer compound raises the probability of bringing the substance to be incorporated in the neighborhood of the water-soluble polymer compound chain. In addition, the water-soluble polymer compound chain has reactive functional groups at the terminal while the catalytic water-soluble polymer compound does not have such reactive functional groups, and thus the force repulsive to the substance to be incorporated is weaker in the water-soluble polymer compound chain than in the catalytic water-soluble polymer compound. In this manner, the substance to be incorporated seems to react with the reactive functional group of the water-soluble polymer compound chain at high bonding ratio. It should be understood that this assumption does not limit the scope of the invention.

### EXAMPLES

The present invention now will be further illustrated by, but is by no means limited to, the following Examples.

### EXAMPLE 1

### (1) Synthesis of acetal-PEG-PLA-methacryloyl

Into a reaction vessel containing 40 mL of tetrahydrofuran (THF) as a solvent at room temperature under an argon atmosphere, were added 0.32 mL (2 mmol) of 3,3'-diethoxy-1-propanol and then 6.2 mL (2 mmol) of 0.3263 mol/L potassium naphthalene THF solution, and the resulting mixture was stirred for 15 minutes for metalation. Additionally, 12 mL (240 mmol) of ethylene oxide was added via a cooled syringe, and the resulting mixture was stirred at room temperature for 2 days to conduct a ring-opening polymerization. Then, 84 mL (84 mmol) of 1 mol/L DL-lactide THF solution was added, and polymerized at room temperature. for 3 hours. Additionally, 4.5 mL (28 mmol) of anhydrous methacrylic acid was added. After the mixture was stirred at room temperature for 2 days, the polymerization was terminated.

The block copolymer solution thus obtained was poured into 2-propanol previously cooled to -15°C, and the precipitated polymer was centrifuged (6000 rpm, 40 minutes, -10°C) to remove the solvent. Theseafter the block copolymer was further purified by repeating this procedure twice, being dissolved in benzene, and lyophilized.

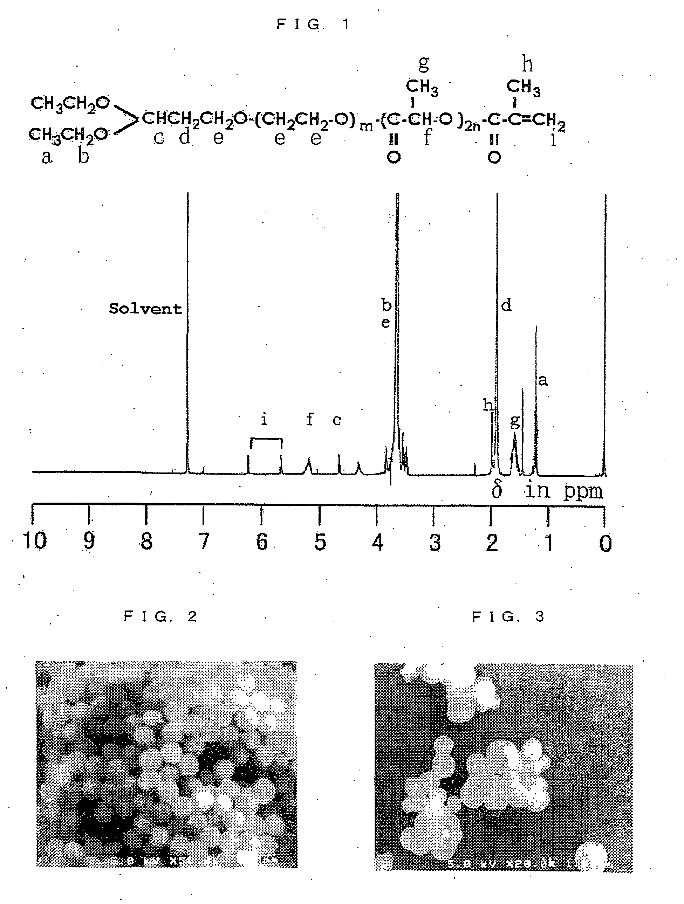

Figure 1 shows the ¹H-NMR spectrum of the block copolymer. The molecular weight of polyethyleneglycol (PEG) was calculated based on the result of GPC measurement. The molecular weight of polylactic acid (PLA) was calculated from the result of ¹H-NMR, together with the molecular weight of PEG obtained by the GPC measurement above. The PEG had a molecular weight of approximately 5,000 and the PLA approximately 500.

### (2) Preparation of particle having aldehyde-terminal-PEG

Into a reaction vessel containing 400 mL of ultrapure water, in which argon replacement had been previously performed, at room temperature under an argon atmosphere, a solution of 388 mg of azobisisobutyronitrile (AIBN) and 8.6 g of the block copolymer [prepared in EXAMPLE 1(1)] in 27 mL of styrene was, after deaeration by bubbling argon, added dropwise while stirring (400 rpm).

After stirring at room temperature for 30 minutes, the solution was further stirred at 60°C for 18 hours and additionally at 80°C for 6 hours (400 rpm) to complete polymerization. After the polymerization reaction, filtration was carried out through a filter paper [Filter paper 2 (diameter: 185 mm); Advantec] to give particles having acetal groups in the surface layer (acetal functionalized particles). The dispersion containing the particles was adjusted to pH 2.0 with 1 mol/L HCl and stirred for 2 hours. Subsequently, the dispersion was 'made to pH 5.0 with 1 mol/L NaOH to deprotect the protecting acetal groups and introduce aldehyde groups to the surface area.

Then, for demineralization, 100 mL of the particle dispersion was dialyzed against 2 L of distilled water for 1 day [molecular weight cut off (MWCO): 12,000 to 14,000; distilled water exchanged 4 times], and filtered through a filter paper [Filter paper 2 (diameter: 185 mm); Advantec] to give particles having aldehyde groups in the surface layer (aldehyde functionalized particles), i.e., aldehyde- terminal-PEG coated particles (particle diameter: 100 nm). The scanning electron microscope (SEM) photograph of the particle (molecular weight of the PEG: approximately 5,000; that of PLA: approximately 500) thus obtained is shown in Figure 2.

In addition, by repeating the procedures of EXAMPLES 1(1) and 1(2) except that anhydrous methacrylic acid was added without using the DL-lactide THF solution after the ring-opening polymerization of ethylene oxide in EXAMPLE 1(1), comparative particles having a PLA unit of a different length (molecular weight of PEG: approximately 5,000; that of PLA: 0) were prepared. The SEM photograph of the comparative particle is shown in Figure 3.

### (3) Binding BSA to aldehyde-terminal-PEG coated particle in the presence of PEG

Into 0.5 mL of a suspension (40 mg/mL, distilled water) of the aldehyde-terminal-PEG coated particles (particle diameter: 100 nm) prepared in EXAMPLE 1(2), PEG 6000 (50 mg) was added and dissolved. To the liquid mixture was added 10 mg of bovine serum albumin (BSA) dissolved in 0.5 mL of 1 mol/L carbonate buffer solution (pH 9.5), and the mixture stirred and reacted at room temperature for 1 hour. Subsequently, to the reaction solution was added 12 mg of NaCNBH₃, and the mixture was stirred and reacted at room temperature overnight. To the reaction solution was added 0.5 mL of 1 mol/L glycine-NaOH buffer solution (pH 8.6), the mixture was stirred at room temperature for 4 hours for blocking, and applied onto a Sepharose CL-6B column (2.5 cm × 40 cm) previously equilibrated with a 0.3 mol/L aqueous sodium chloride solution and eluted into 2 mL fractions. Peak fractions containing particles first eluted were collected.

### COMPARATIVE EXAMPLE 1

### (1) Binding BSA to aldehyde-terminal-PEG coated particles in the absence of PEG

By repeating the procedures of EXAMPLE 1(3) except that PEG 6000 was not added to the suspension of the aldehyde-terminal-PEG coated particles, peak fractions containing particles first eluted from the Sepharose CL-6B column were collected.

### EVALUATION EXAMPLE 1

### (1) Quantitative determination of BSA on the surface of particles

In this evaluation example, the protein quantification method established by the present inventors was used, i.e., a method of quantifying the amount of protein on the surface of particles by using a commercially available BCA-protein assay reagent (Pierce), and in addition, raising the sensitivity by increasing the amount of sample.

More specifically, dilution series of BSA as a standard substance in the range of 0 to 100 µg/mL in 0.3 M aqueous sodium chloride solution were prepared, and to 50 µL of each dilution series was added 200µL of a BCA-protein assay reagent, and the resulting solution was sealed and allowed to stand at 37°C for 30 minutes. After the reaction, the absorbance of the solution was measured at 562 nm using a microcell allowing measurement of small amount of samples with a light path of 1 cm.

The fractions containing the BSA-bonding particles first eluted from the column prepared in EXAMPLE 1 and COMPARATIVE EXAMPLE 1 respectively were used as the samples. To 50 µL thereof was added 200 µL of the BCA-protein assay reagent, in a manner similar to the examples above, the mixture was sealed and allowed to stand at 37°C for 30 minutes, and the absorbance thereof was measured at 562 nm. The factions containing the particles were clouded and had a significant absorbance at 562 nm from the beginning due to a scattering of light, and thus the measured values of samples were calculated by subtracting from actual measured values the absorbance obtained when 200 µL of distilled water.was used, replacing the BCA reagent; and the mass of proteins was calculated by comparing the measured value with the absorbance obtained with the dilution series of the standard substance.

The BSA-bonding particle prepared in EXAMPLE 1 contained 16.8 µg of BSA per 1 mg of particles, while that prepared in COMPARATIVE EXAMPLE 1 contained 3.5 µg of BSA per 1 mg of particles. It was confirmed that the presence of 5% PEG 6000 in the reaction mixture raised the amount of BSA bonded by as much as 4.8 times.

### EXAMPLE 2

### (1) Effect of the presence of PEG on reaction with an antibody F(ab')₂ fraction

Mixed particle/PEG dispersion liquids were prepared by adding PEG 6000 in an amount respectively of 0 mg, 20 mg, 40 mg, and 60 mg to 0.5 mL of PEG 6000 to the suspensions (40 mg/mL, distilled water) of the aldehyde-terminal-PEG coated particles (particle diameter: 100 nm) prepared in EXAMPLE 1. To each suspension liquid, 1 mg of anti-C-reactive protein (CRP) rabbit antibody F(ab')₂ fraction previously dialyzed against 0.5 mL of 1 mol/L carbonate buffer solution (pH 9.5) was added, and the mixture reacted at room temperature for 1 hour. The anti-CRP rabbit antibody F(ab')₂ fraction above was prepared according to a common method from an anti-CRP rabbit antibody (Dako). To the reaction solution 12 mg of NaCNBH₃ was added, and additionally the mixture stirred at room temperature overnight.

Blocking was carried out by adding 0.5 mL of 1 mol/L glycine-NaOH buffer solution (pH 8.6) to the reaction solution and stirring at room temperature for 4 hours, and the mixture applied onto a Sepharose CL-6B column (2.5cm×40cm) previously equilibrated with 0.3 mol/L aqueous sodium chloride solution and eluted into 2 mL fractions. Peak fractions containing particles first eluted from the column were collected.

The mass of the proteins on the particle collected was determined according to the method described in Evaluation Example 1, and the results are summarized in TABLE 1. As apparent from TABLE 1, when the PEG concentration during the reaction is 0% (i.e., in the absence of PEG) or 6%, the amount of proteins bonded is less than the detectable amount, and the amount of proteins bonded is largest in the presence of PEG 6000 in a concentration of 4%, indicating that the most suitable concentration is 4% in the case of antibodies.

**TABLE 1**

| PEG Conc. (%) | Amount of Antibody Bonded (µg/mg) |
|---|---|
| 0 | 0 |
| 2 | 0.78 |
| 4 | 1.45 |
| 6 | 0 |

### INDUSTRIAL APPLICABILITY

The method of the present invention allows the bonding of a substance to be incorporated (for example, biological molecules) to the terminals of a water-soluble polymer compound (particularly PEG) at high efficiency, in reactions of reacting reactive functional groups present at the terminals of the polymer compound which is formed in the manner of bristles of a brush on the surface of a base material (for example, polymer brush) with the substance to be incorporated. The method of the present invention is monumental in the sense that it allows a bonding of biological molecules to PEG molecules, which are gathering attention as one of the functional molecules, and thus leads to an expansion of the application area. In addition, the method of the present invention allows not only an introduction of proteins to PEG terminals of PEG brush particles but also to the introduction of biomolecules to the surface of a variety of medical devices, and thus enables the creation of high-performance biological functional interfaces.

Although the present invention has been described with reference to specific embodiments, various changes and modifications obvious to those skilled in the art are possible without departing from the scope of the appended claims.

## Claims

1. A method of bonding a substance to be incorporated to a free terminal of a water-soluble polymer compound chain, **characterized by** reacting
(1) a reactive functional group present at the free terminal of the water-soluble polymer compound chain which is bonded at a binding terminal thereof in the manner of bristles of a brush onto a surface of a base material; with
(2) the substance to be incorporated capable of reacting with the reactive functional group;
in the presence of a water-soluble polymer compound which promotes the bonding.

2. The method according to claim 1, wherein the water-soluble polymer compound chain bonded in the manner of bristles of a brush onto a surface.of a base material is substantially made of polyethyleneglycol.

3. The method according to claim 1 or 2, wherein the water-soluble polymer compound which promotes the bonding is polyethyleneglycol.

4. The method according to any one of claims 1 to 3, wherein the substance to be incorporated capable of reacting with the reactive functional group is a protein.

5. The method according to any one of claims 1 to 3, wherein the substance to be incorporated capable of reacting with the reactive functional group is a DNA.

6. The method according to any one of claims 1 to 3, wherein the substance to be incorporated capable of reacting with the reactive functional group is a cell.

7. The method according to any one of claims 1 to 6, wherein the reactive functional group is an aldehyde group.

8. The method according to any one of claims 1 to 7, wherein the base material is a latex particle.

9. The method according to any one of claims 1 to 7, wherein the base material is a polymer micelle.

10. A method of bonding a substance to be incorporated into a free terminal of a water-soluble polymer compound chain, **characterized by** reacting
(1) a core-shell particle consisting of
(a) a core portion substantially made of a water-insoluble polymer compound, and
(b) a shell portion substantially made of a water-soluble polymer compound having a reactive functional group, and covering a surface of the core portion in the manner of bristles of a brush,
the core portion and the shell portion being, as a whole, a block copolymer of a water-insoluble polymer and a water-soluble polymer; with
(2) the substance to be incorporated capable of reacting with the reactive functional group;
in the presence of a water-soluble polymer compound which promotes the bonding.
